# EUROPÄISCHE PATENTSCHRIFT

(11) **EP 1 651 308 B1**
(45) Veröffentlichungstag und Bekanntmachung des Hinweises auf die Patenterteilung: **11.05.2011**
(21) Anmeldenummer: 04734260.5
(22) Anmeldetag: 21.05.2004
(51) Int. Cl.: A61N 1/05, A61L 31/16, A61L 31/10, A61L 31/04, A61L 26/00, A61K 47/48, C08L 5/08, C08B 37/00

(54) **IMPLANTIERBARE STIMULATIONSELEKTRODE MIT EINER BESCHICHTUNG ZUR ERHÖHUNG DER GEWEBSVERTRÄGLICHKEIT**
IMPLANTABLE STIMULATION ELECTRODE WITH A COATING FOR INCREASING TISSUE COMPATIBILITY
ELECTRODE DE STIMULATION IMPLANTABLE POURVUE D'UN REVETEMENT DESTINE A AUGMENTER LA COMPATIBILITE TISSULAIRE

(30) Priorität: 21.06.2003 DE 10328816
(43) Veröffentlichungstag der Anmeldung: 03.05.2006
(73) Patentinhaber: BIOTRONIK SE & Co. KG, 12359 Berlin (DE)
(72) Erfinder: BAYER, Gerd, 91054 Erlangen (DE); BORCK, Alexander, 91086 Aurachtal (DE)
(74) Vertreter: Lindner-Vogt, Karin L.
(86) Internationale Anmeldenummer: PCT/EP2004/005550
(87) Internationale Veröffentlichungsnummer: WO 2004/112891

(56) Entgegenhaltungen:
- WO-A-98/12243
- US-A- 5 820 917
- US-A- 5 866 113
- US-A1- 2003 091 609
- US-A1- 2003 091 827

## Beschreibung

Die Erfindung betrifft eine implantierbare Stimulationselektrode mit einer Beschichtung zur Erhöhung der Gewebsverträglichkeit mit den im Oberbegriff des Anspruchs 1 genannten Merkmalen.

Implantierbare Elektroden zur Stimulation von Körpergewebe, insbesondere zur Verwendung in Herzschrittmachern, Defibrillatoren, Knochen-oder Neurostimulatoren, sind in großer Vielgestaltigkeit bekannt. Der weit überwiegende Anteil derartiger Stimulationselektroden basiert auf metallischen Werkstoffen, da diese zur Übertragung elektrischer Ströme an lebendes Gewebe auf Grund ihrer guten Leitfähigkeit prädestiniert sind. Andere Lösungen sehen den Einsatz von leitfähigen Polymeren vor (z. B. US 5,080,099).

Für den Gebrauchswert einer implantierbaren Stimulationselektrode - insbesondere einer solchen, die für den Langzeiteinsatz an einem Gewebestimulator mit einer erschöpfbaren Energiequelle gedacht ist und daher zum minimalen Energieverbrauch beitragen muss - sind eine hohe Elektrodenkapazität und damit niedrige Elektrodenimpedanz und ein möglichst hoher Grad an Biokompatibilität von herausragender Bedeutung.

So wurde etwa in der EP 0 453 117 A1 und der WO 93/02739 eine hochentwickelte implantierbare Stimulationselektrode beschrieben. Die Elektrode besteht aus einem mehrschichtigen, aus Faser- oder Drahtmaterial gepressten Platingrundkörper, einer Haftschicht, einer Pt-, C-oder AI-Texturierungsschicht mit rauer Oberfläche und einer katalytisch wirkenden Pt- oder Pt/C-Deckschicht. Die Stimulationselektrode weist ferner eine sehr große aktive Oberfläche mit fraktaler Oberflächenstruktur auf und kann wahlweise auch in Form eines Titangrundkörpers mit einer Iridium-, Iridiumnitrid- oder Iridiumoxidbeschichtung realisiert werden.

In den ersten Wochen nach der Implantation von Stimulationselektroden ist allgemein eine temporäre Reizschwellenerhöhung festzustellen, die sich auf lokale Entzündungserscheinungen des anliegenden Gewebes zurückführen lassen. Diese Entzündungserscheinungen führen außerdem zu einem ungünstigen Einwachsverhalten der Stimulationselektroden, was langfristig die Stimulationseigenschaften des Systems negativ beeinflusst.

Aus der DE 196 30 563 ist eine implantierbare Stimulationselektrode der gattungsgemäßen Art bekannt. Die dort beschriebene Stimulationselektrode zeigt insbesondere eine erhöhte Gewebsverträglichkeit. Dies wird dadurch erreicht, dass eine im wesentlichen die gesamte äußere Oberfläche der Stimulationselektrode bildende, dünne, spezifisch funktionalisierte organische Beschichtung vorgesehen ist, die auf Grund irreversibler Physisorption oder kovalenter Bindung an der darunter liegenden Oberfläche fest haftet. Als Beschichtungsmaterialien werden u.a. Silane und synthetische Polymere wie Polystyrensulfonat, Polyvinylsulfonat oder Polyallylamin vorgeschlagen. Die organische Beschichtung kann auch mehrschichtig sein, wobei an der äußeren Oberfläche insbesondere Polyethylenoxid oder Polyethylenglycol terminiert ist. Ferner wird angesprochen, dass die organische Schicht einen medizinischen Wirkstoff, insbesondere ein entzündungshemmendes Medikament beinhaltet, der aus der organischen Beschichtung diffusions- oder lösungsgesteuert austragbar ist.

Die geschilderten Verbesserungen durch die Beschichtung der Stimulationselektrode führen zwar bereits zu einer deutlichen Minderung der temporären Reizschwellenerhöhung, sind aber relativ aufwendig und damit kostspielig in der Umsetzung und fordern wegen der synthetischen Natur der verwendeten Materialien umfangreiche Tests zur Evaluierung der Biokompatibilität. Weiterhin ist es im Falle des gewünschten Zusatzes von entzündungshemmenden Wirkstoffen notwendig, die Materialeigenschaften der Wirkstoffe und der sie einbettenden organischen Beschichtung durch umfangreiche Tests aufeinander abzustimmen.

Aufgabe der vorliegenden Erfindung ist es, eine Beschichtung für eine implantierbare Stimulationselektrode bereitzustellen, die Gewebsirritationen nach der Implantation und speziell einen damit einhergehenden Reizschwellenanstieg vermeidet. Die Beschichtung soll eine sehr hohe Biokompatibilität besitzen und zudem von sich aus entzündungshemmend wirken. Ferner soll die Beschichtung aus möglichst wenig Komponenten bestehen, so dass die Herstellung vereinfacht wird.

Diese Aufgabe wird durch die implantierbare Stimulationselektrode nach Anspruch 1 gelöst.

Die implantierbare Stimulationselektrode weist eine gewendelte Zuleitung mit einer elektrisch isolierenden Ummantelung und einen mit einer Beschichtung zur Erhöhung der Gewebsverträglichkeit versehenen metallischen Grundkörper auf, welcher am distalen Ende der Zuleitung befestigt ist und auf dem eine oder mehrere Zwischenschichten aus Iridiumnitrid (IrN) mit einer fraktalartig vergrößerten Oberfläche aufgebracht sind. Die Zwischenschicht(en) sind mit einer Polysaccharidschicht aus Hyaluronsäure und/oder Hyaluronsäure-Derivaten mit einer Schichtdicke zwischen 10 und 400 µm bedeckt, die durch Physisorption oder kovalente Bindung an der darunter liegenden Zwischenschicht anhaftet. Die Beschichtung umfasst eine Polysaccharidschicht aus Hyaluronsäure und/oder Hyaluronsäure-Derivaten. Es hat sich überraschenderweise gezeigt, dass die Aufbringung einer solchen Polysaccharidschicht zu keiner nennenswerten Erhöhung der Elektrodenimpedanz führt und demnach kaum oder gar nicht Einfluss auf den Energieverbrauch des Stimulationssystems hat. Ferner zeichnen sich Hyaluronsäure und seine Derivate durch ihre sehr gute Biokompatibilität aus, da die Materialien natürlichem Ursprungs sind. Weiterhin hat es sich gezeigt, dass Hyaluronsäure als auch seine Derivate eine eigenständige entzündungshemmende Wirkung besitzen und damit wirkungsvoll Gewebsirritationen verhindert oder zumindest stark vermindert werden können.

Hyaluronsäure (Hyaluronan) ist ein einfaches Glykosaminoglykan der extrazellulären Matrix. Es wird an der Oberfläche von Fibroblasten synthetisiert und kommt als einziges Glykosaminoglykan nicht als Proteoglykan vor. Hyaluronsäure ist eine hochmolekulare Verbindung mit M_{R} zwischen 50.000 und mehreren Millionen. Grundbaustein der Hyaluronsäure ist ein aus D-Glucuronsäure und N-Acetyl-d-glucosamin in β1-3-glykosidischer Bindung aufgebautes Aminodisaccharid, das mit der nächsten Einheit β1-4-glykosidisch verbunden ist:

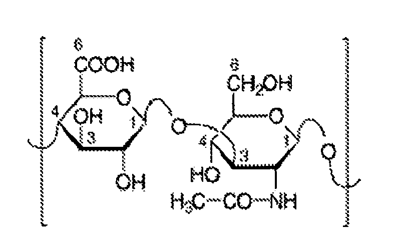

Die unverzweigte Kette der Hyaluronsäure besteht aus 2.000-10.000 solcher Einheiten. Durch Hyaluronidasen werden β-glykosidische Bindungen hydrolysiert und so die Hyaluronsäure zu kleineren Bruchstücken abgebaut. Die - meist als Kalium-Salz - im Handel befindliche Hyaluronsäure ist aus menschlichen Nabelschnüren oder Hahnenkämmen isoliert, wird aber zunehmend biotechnologisch durch bakterielle Fermentation hergestellt.

Zur Modifizierung von Hyaluronsäure, d.h. Darstellung von Hyaluronsäure-Derivaten, werden literaturbekannte Verfahren eingesetzt (z. B. Danishefsky, Arch. Biochem. Biophys., 90, 1960, S. 114 ff.; Nagasawa, Carbohydr. Res., 58, 1977, S. 47 ff.; Ayotte, Carbohydr. Res. 145, 1986, S. 267 ff.; Ogamo, Carbohydr. Res. 193, 1989, S. 165 ff.; Jesaja, Can. J. Chem.; 67, 1989, S. 1449 ff.; Mulloy, Carbohydr. Res. 255, 1994, S. 1 ff.). Dabei handelt es sich um regio- und stereoselektive und nicht regio-und stereoselektive (statische) Reaktionen. Basierend auf diesem Verfahren kann Hyaluronsäure insbesondere durch N- und O-Desulfatierung, O-Desulfatierung, 6-O-Desulfatierung, Deacetylierung oder Acetylierung sowie Sulfatierung, Acylierung mit aliphatischen oder aromatischem Rest verändert werden. Insbesondere können durch die bekannten Verfahren Aminogruppen, Sulfat- oder Carboxylreste unter Anwendung von Schutzgruppenchemie und bekannten, zum Teil regioselektiven Reaktionen der organischen Chemie eingeführt werden.

Unter dem Begriff "Hyaluronsäue-Derivate" im Sinne der Erfindung werden demnach alle durch gezielte Modifizierungen der natürlichen Hyaluronsäure strukturell zum Ausgangsprodukt veränderten Reaktionsprodukte verstanden. Unter dem Begriff "Hyaluronsäure und Hyaluronsäure-Derivate" werden ferner alle polyelektrolytischen Salze derselben, z.B. Natrium-, Kalium-, Magnesium- und Kalziumsalze, verstanden. Als "Modifizierungen" im erfindungsgemäßen Sinne werden die aufgeführten und weiteren bekannten Reaktionen der organischen Chemie zur Umsetzung der funktionellen Gruppen der Hyaluronsäure angesehen.

Hyaluronsäure und die Hyaluronsäure-Derivate können als Einzelsubstanzen, Co- oder Blockpolymere aus Hyaluronsäure und Hyaluronsäure-Derivaten, als auch in Form von Mischungen der vorgenanten Einzelsubstanzen und Polymere kovalent und/oder durch Physisorption an der Stimulationselektrodenoberfläche immobilisiert werden.

Eine kovalente Anbindung der Polysaccharidschicht an die Zwischenschicht des metallischen Grundkörpers der Stimulationselektrode erfolgt vorzugsweise durch Einpunkts- oder Mehrpunktsaufhängung an Spacer. Weiterhin wird vorzugsweise durch Vernetzung einer zuvor aufgebrachten (primären) Polysaccharidschicht eine mechanische und/oder chemische Stabilisierung des Beschichtungsmaterials gegen enzymatischen und hydrolytischen Abbau als auch gegen mechanischen Stress erreicht. Die Immobilisierung der Polysaccharidschicht auf Zwischenschicht des metallischen Grundkörpersder Stimulationselektrode kann nach bekannten Methoden der Immobilisierung von Enzymen, Methoden der Membranherstellung, Kunststoffverarbeitung, Polymerchemie, der Peptid-, Protein- und Zuckerchemie über kovalente Bindungen mit und ohne Verwendung von Spacern, mittels Einpunkts- und Mehrpunktaufhängung, Endpunktaufhängung als Mono- oder Multilayer oder mit zusätzlicher Stabilisierung durch Quervernetzung erfolgen.

Als vorteilhaft hat sich eine Beschichtung mit einer Schichtdicke im Bereich zwischen 10-400 µm, insbesondere 50-120 µm, erwiesen. Bei den genannten Schichtdicken konnte noch kein signifikanter Effekt auf die Elektrodenimpedanz der Stimulationselektrode festgestellt werden.

Weiterhin ist bevorzugt, wenn die Hyaluronsäure oder die Hyaluronsäure-Derivate nach Sterilisation noch ein durchschnittliches Molekulargewicht im Bereich von ca. 300.000-500.000, insbesondere 380.000-420.000 Dalton aufweisen. Im beanspruchten Molekulargewichtsbereich erreicht die eigenständige therapeutische Wirkung der Hyaluronsäure und seiner Derivate ein Maximum (Papakonstantinou, G. Karakiulakis, O. Eickelberg, A.P. Perruchoud, L.H. Block, and M. Roth ; A 340 kDa hyaluronic acid secreted by human vascular smooth muscle cells regulates their proliferation and migration, Glycobiology 1998, 8, 821-830).

Ein weiterer vorteilhafter Aspekt der erfindungsgemäßen Lehre liegt in der gezielten Beeinflussung des in vivo Degradationsverhalten des Biopolymers. Unter dem Begriff "Degradationsverhalten" wird der durch chemische, thermische, oxidative, mechanische oder biologische Prozesse stattfindende Abbau der Polysaccharidschicht im lebendem Organismus über die Zeit verstanden. Einerseits soll sichergestellt werden, dass zumindest in den ersten Wochen nach der Implantation lokale Entzündungserscheinungen des anliegenden Gewebes gelindert oder gar vermieden werden. Andererseits soll die Beschichtung über einen bestimmten Zeitraum eine Oberflächenadsorption von hochmolekularen Biomolekülen auf der Elektrodenoberfläche verhindern oder zumindest deutlich zurückdrängen, da ansonsten mittel- und langfristig ebenfalls mit einem Anstieg der Elektrodenimpedanz zu rechnen ist.

Vorzugsweise ist die Polysaccharidschicht derart beschaffen ist, dass die in vivo Degradation der Polysaccharidschicht von außen in Richtung des Grundkörpers der Stimulationselektrode verlangsamt ist. Das Degradationsverhalten kann dabei kontinuierlich oder sprunghaft verändert werden. Nach letzterer Variante umfasst die Polysaccharidschicht zumindest zwei Teilschichten mit unterschiedlichem Degradationsverhalten, wobei das Degradationsverhalten innerhalb jeder Teilschicht kontinuierlich veränderlich oder konstant über die Teilschicht festlegbar ist.

Die Herstellung derartiger Beschichtungen kann mit Hilfe an sich bekannter Sprüh- und Tauchbeschichtungsverfahren erfolgen.

Vorzugsweise ist die Polysaccharidschicht derart beschaffen, dass ein dem Grundkörper der Elektrode abgewandter, äußerer Bereich der Polysaccharidschicht innerhalb von 100 Tagen in vivo abgebaut wird. Der äußere Bereich ist vorzugsweise 10 bis 250 µm, insbesondere 50 bis 150 µm, dick. Wenn die Polysaccharidschicht aus zumindest zwei Teilschichten mit unterschiedlichem Degradationsverhalten besteht, ist zur Erreichung dieses Ziels eine äußere Teilschicht derart modifiziert, dass sich diese äußere Teilschicht um mehr als 50 Gew% innerhalb von 100 Tagen in vivo abbaut. Die äußere Teilschicht ist vorzugsweise 10 bis 250 µm, insbesondere 50 bis 150 µm, dick.

Es hat sich ferner überraschenderweise gezeigt, dass in Gegenwart der Polysaccharidschicht auch die Oberflächenadsorption von hochmolekularen Biomolekülen auf der Elektrodenoberfläche verhindert oder zumindest deutlich zurückgedrängt ist. Vorzugsweise ist daher die Polysaccharidschicht derart beschaffen, dass ein dem Grundkörper der Elektrode zugewandter, innerer Bereich der Polysaccharidschicht zumindest nicht vollständig innerhalb von zwei Jahren in vivo abgebaut wird. Der innere Bereich ist vorzugsweise 3 bis 50 µm, insbesondere 5 bis 20 µm, dick. Wenn die Polysaccharidschicht aus zumindest zwei Teilschichten mit unterschiedlichem Degradationsverhalten besteht, ist zur Erreichung dieses Ziels insbesondere eine innere Teilschicht, die sich unmittelbar der darunter liegenden Zwischenschicht auf der Oberfläche des Grundkörpers der Stimulationselektrode anschließt, derart modifiziert, dass sich diese innere Teilschicht um nicht mehr als 20 Gew% innerhalb von zwei Jahren in vivo abbaut. Die äußere Teilschicht ist vorzugsweise 3 bis 50 µm, insbesondere 5 bis 20 µm, dick.

Das Degradationsverhalten von Hyaluronsäure und seiner Derivate kann u.a. durch Quervernetzung und reduktive Fixierung beeinflusst werden, wie beispielsweise aus den Dokumenten US 4,582,865, US 5,550,187, US 5,510,121 und WO 00/46252 bekannt. Unter reduktive Fixierung wird die gezielte Umsetzung ungesättigter Funktionalitäten des Polysaccharids mit hydridischen Reduktionsmitteln, wie z.B. Natriumborhydrid, verstanden. Die Quervernetzung kann z.B. mit Hilfe der folgenden Reagenzien durchgeführt werden:
Formaldyhyd, Glutaraldehyd, Divinylsulfon, Polyanhydride, Polyaldehyde, Carbodiimide, Epichlorohydrin, Ethylenglykol-diglycidylether, Butandiol-diglycidylether, Polyglycerol-polyglycidylether, Polyethylenglykoldiglycidylether, Polypropylenglykol-diglycidylether oder bis- oder Polyepoxy-Vernetzer, wie 1,2,3,4-Diepoxybutan oder 1,2,7,8-Diepoxyoctan.

Der Zusammenhang zwischen Vernetzungsgrad, reduktiver Fixierung und Degradationsverhalten kann über herkömmliche Testverfahren ermittelt werden. Ein unterschiedlicher Vernetzungsgrad führt bei ansonsten gleicher Fixierung zu einem unterschiedlichen Quellverhalten der Polysaccharidschicht. Durch Verzicht auf die Fixierung oder nur unvollständige Fixierung wird die Degradation der Polysaccharidschicht beschleunigt. Das Quellverhalten lässt sich u.a. gravimetrisch bestimmen. Weiterhin lässt sich der Vernetzungsgrad und der Umfang der reduktiven Fixierung durch infrarotspektroskopische Analyse an vernetzten Hyaluronsäurefolien bestimmen. Der Bezug zur Degradation kann durch eine GPC Analytik, d.h. durch Molmassenbestimmung degradierter Hyaluronsäure, an Eluenten hergestellt werden.

Der Einfluss der genannten Modifikationen auf das in vivo Degradationsverhalten ist allgemein bekannt. Da das Abbauverhalten aber u.a. auch von weiteren geometrischen und physiologischen Faktoren abhängt, ist in der Regel eine individuelle Anpassung des Systems an die jeweiligen Erfordernisse notwendig.

Die Beschichtung kann in der Regel auf alle bekannten Stimulationselektroden aufgebracht werden. Die dünne Polysaccharidschicht aus Hyaluronsäure und/oder Hyaluronsäure-Derivaten wird dazu mittels gängiger Sprühverfahren oder aus der Lösung abgeschieden.

Die prinzipielle Herstellung einer kovalent anhaftenden Polysaccharidschicht wird in der WO 00/56377 beschrieben. Eine Substratoberfläche wird dazu mit reaktiven Funktionalitäten modifiziert, aktivierte Hyaluronsäure wird bereit gestellt und diese wird dann unter geeigneten Bedingungen kovalent an die reaktiven Funktionalitäten gebunden. In eben gleicher Weise lässt sich die Polysaccharidschicht an die Oberfläche der Stimulationselektrode binden.

Weiterhin offenbart die bereits erwähnte DE 196 30 563 ein Verfahren zur Verbesserung der Haftung einer Beschichtung infolge verstärkter Physisorption bzw. kovalenter Bindung. In einem ersten Schritt wird eine reaktive Funktionalität auf der Substratoberfläche erzeugt. Die reaktive Funktionalität umfasst insbesondere Amine, Aldehyde, Sulfide, Alkohole, Säurehalogenide und Isocyanate. An die genannte Funktionalität kann dann - unter Rückgriff auf an sich bekannte Kopplungsverfahren - die Polysaccharidschicht kovalent gebunden werden.

Nach einem weiteren Aspekt der vorliegenden Erfindung wird die schon bestehende eigenständige therapeutische Wirkung der Hyaluronsäure durch weitere Wirkstoffe, die in die Beschichtung eingebettet sind und durch den allmählichen Abbau der Beschichtung bzw. Diffusion in das umliegende Gewebe freigesetzt werden, ergänzt. Es hat sich gezeigt, dass insbesondere die entzündungshemmenden Steroide Dexamethason und/oder Dexamethasonnatriumphosphat (DMNP) in einer zur Entfaltung einer pharmakologischen Wirkung ausreichenden Konzentration besonders hierfür geeignet sind, da sie nachweislich zur Stabilisierung der am Implantat anliegenden Macrophagen beitragen und damit die Langzeitverträglichkeit der Beschichtung verbessern.

Überraschenderweise hat es sich zudem gezeigt, dass Dexamethason und/oder DMNP einen positiven Effekt auf die Phasengrenzkapazität der Elektrode hat. Die durch die Beschichtung zwar nur unwesentlich erhöhte Phasengrenzkapazität wurde durch die Anlagerung von Dexamethason und/oder DMNP nahezu auf den ohne Polysaccharidschicht messbaren Wert gemindert.

Weiterhin ist bevorzugt, wenn die Polysaccharidschicht eine Haftvermittlerschicht aus Chitosan umfasst. Die Haftvermittlerschicht schließt sich unmittelbar der Zwischenschicht auf dem Grundkörper an. Es hat sich überraschenderweise gezeigt, dass in Gegenwart einer solchen Haftvermittlerschicht sehr gleichmäßige und stark haftende Beschichtungen erzeugt werden können. Zudem ist Chitosan Werkstoff natürlichem Ursprungs und damit gut bioverträglich. Die als Haftvermittlerschicht ist vorzugsweise 0,1 bis 50 µm, insbesondere 1 bis 10 µm, dick und kann ebenso wie die Hyaluronsäure und ihre Derivate zur Beeinflussung Ihres Degradationsverhaltens modifiziert werden. Insbesondere kann die Haftvermittlerschicht derart ausgebildet sein, dass sie als innere Teilschicht oder innerer Bereich der Polysaccharidschicht im oben genannten Sinne agieren kann. Eine signifikante Änderung der elektrischen Übertragungseigenschaften der Elektrode durch die Haftvermittlerschicht wurde nicht festgestellt.

Nach einer weiteren bevorzugten Variante der Erfindung beinhaltet die Polysaccharidschicht zumindest in Teilbereichen oder Teilschichten Chitosan. Hierdurch kann das Haftvermögen der Polysacharidschicht weiter verbessert werden und es können auch auf den sehr komplexen Geometrien des Substrats gleichmäßige Beschichtungen erzeugt werden. Die Stabilität der Polysacharidschicht kann gesteigert werden, wenn durch Quarternisierung der aminischen Funktionen des Chitosans polykationische Ladungen erzeugt werden. Werden Hyaluronsäure und seine Derivate als polyanionische Präperate zugemengt, so bildet sich ein Symplexgel. Die schon sehr starke Ion/Ion-Wechselwirkung zwischen den Komponenten kann durch Quervernetzung weiter erhöht werden. Ein Gewichtsanteil des Chitosans am Gesamtgewicht der Polysaccharidschicht beträgt vorzugsweise nicht mehr als 50%.

Nachfolgend wird die Erfindung anhand von Ausführungsbeispielen und der dazugehörigen Zeichnungen näher erläutert. Es zeigen:
- Fig. 1: Einen Querschnitt sowie einen vergrößerten Ausschnitt hier- aus vom distalen Ende einer unipolaren Herzschrittmacher- Elektrodenleitung mit einem Elektrodenkopf gemäß einer Ausführungsform der Erfindung und
- Fig. 2: eine Prinzipdarstellung zur Erläuterung des Aufbaus einer implantierbaren Stimulationselektrode gemäß einer Ausfüh- rungsform der Erfindung.

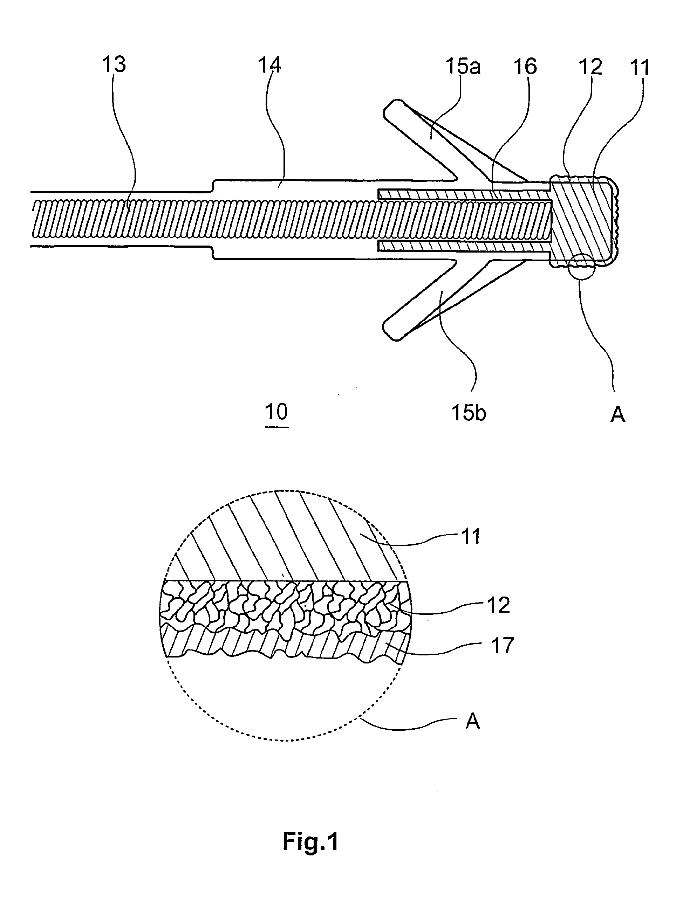

Die Fig. 1 zeigt schematisch einen Querschnitt und einem vergrößerten Ausschnitt A ein distales Ende einer unipolaren Herzschrittmacher-Elektrodenleitung für eine Noppenelektrode 10 mit einem zylinderförmigen Grundkörper 11 aus Titan. Der zylinderförmige Grundkörper 11 weist eine aus Iridiumnitrid (IrN) bestehende Oberflächenbeschichtung 12 (Zwischenschicht) auf, die mittels Kathodenzerstäubung (reaktives Sputtern) auf dem Grundkörper 11 in bekannter Weise aufgebracht ist. Die Elektrode umfasst ferner eine gewendelte, elektrisch leitende Zuleitung 13 mit einer elektrisch isolierenden Ummantelung 14 aus Silikon. An die Silikonummantelung 14 angeformt sind proximal gerichtete Finnen 15a und 15b, welche zur Fixierung der Elektrode 10 im Herzen dienen, wobei die Oberfläche des Grundkörpers 11 in Kontakt mit einer - hier nicht dargestellten - Herzinnenwandung gehalten wird. Der Grundkörper 11 ist mittels eines hohlzylindrischen Ansatzes 16 über die Zuleitung 13 geschoben und dort befestigt.

Der in Fig. 1 dargestellte Ausschnitt A zeigt stark schematisiert und vergrößert einen Schnitt durch eine aktive Oberfläche des Grundkörpers 11 sowie dessen Oberflächenbeschichtung 12 und eine sich daran anschließende Beschichtung 17 zur Erhöhung der Gewebsverträglichkeit, die zumindest eine Polysaccharidschicht aus Hyaluronsäure und/oder Hyaluronsäure-Derivaten unfasst. Eine Oberfläche der Iridiumnitrid-Beschichtung 12 ist herstellungsbedingt fraktalartig vergrößert, d. h. infolge geeigneter Wahl der Verfahrensparameter des Sputterverfahrens beträgt die Oberflächenvergrößerung zwei bis über drei Größenordnungen, bezogen auf die Oberfläche eines glatten Zylinders mit den Abmessungen des Grundkörpers 11. An die Iridiumnitrid-Beschichtung 12 ist - wie im folgenden noch näher erläutert wird - die nur wenige bis einige hundert Mikrometer dicke Polysaccharidschicht gebunden, wobei die elektrischen Eigenschaften der Elektrode 10 praktisch nicht beeinträchtigt werden, aber der Reizschwellenverlauf während des Einwachsens der Elektrode 10 an der Herzwand deutlich positiv beeinflusst wird.

### Ausführungsbeispiel 1 - Kovalente Anbindung

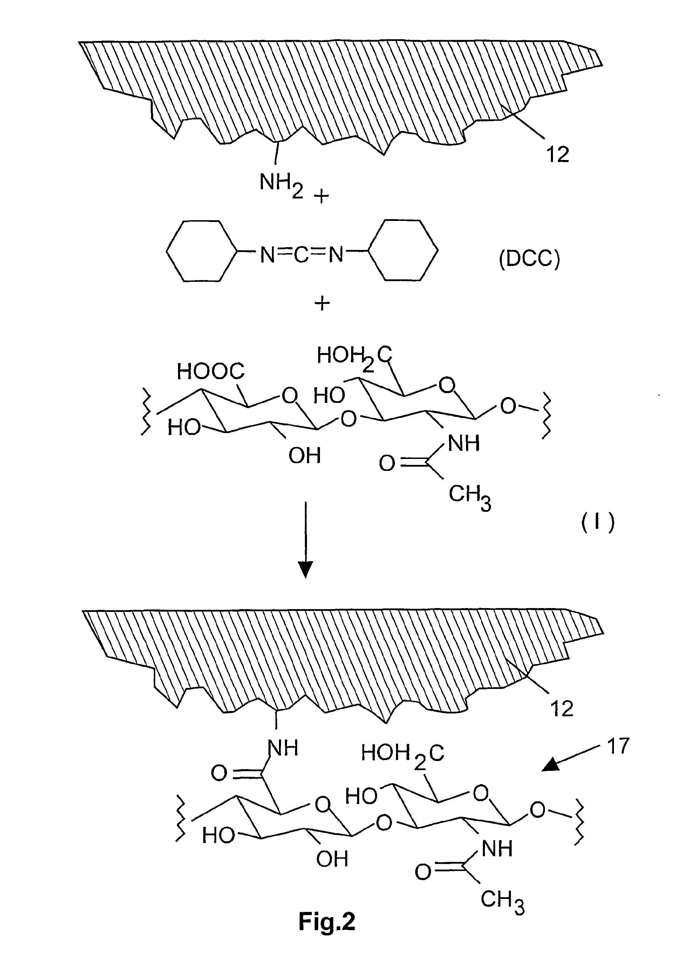

Die Fig. 2 offenbart in einer Prinzipdarstellung den Aufbau und die Darstellung einer Beschichtung 17 aus Hyaluronsäure, wobei diese kovalent an die darunter liegende Oberfläche, d.h. im konkreten Fall die Iridiumnitrid-Beschichtung 12, gebunden wird. Alternativ oder ergänzend kann die Bindung durch Physisorption der Hyaluronsäure auf der Iridiumnitrid-Beschichtung 12 erfolgen. Unter Physisorption wird jedwede elektrostatische Wechselwirkung zwischen der Oberfläche der Iridiumnitrid-Beschichtung 12 und der Hyaluronsäure (I), insbesondere Van-der-Waals-Wechselwirkung, verstanden.

In einem ersten - hier nicht dargestellten - Verfahrensschritt findet eine Aminierung der Iridiumnitrid-Oberfläche 12 statt. Hierzu kann auf zahlreiche bekannte Verfahren zurückgegriffen werden, wobei primäre oder sekundäre Amine, vorzugsweise aber primäre Amine, auf der Oberfläche der Iridiumnitrid-Beschichtung 12 fixiert werden. So bietet sich insbesondere eine Plasmaaktivierung in Gegenwart von Aminen, z. B. N-Heptylamin oder anderen aliphatischen oder aromatischen Aminen, an. Aufgrund der allgemeinen Bekanntheit, Zugänglichkeit und Variabilität derartiger Verfahren wird auf Einzelheiten bei der Anbindung von Funktionalitäten auf die Iridiumnitrid-Beschichtung 12 verzichtet. Festzuhalten bleibt lediglich, dass nach Beendigung des ersten Verfahrensschrittes reaktive Funktionalitäten - hier beispielhaft ein primäres Amin - an der Oberfläche Iridiumnitrid-Beschichtung 12 gebunden sind.

In einem sich anschließenden, zweiten Verfahrensschritt erfolgt eine kovalente Anbindung von Hyaluronsäure (I) analog der aus der Peptidsynthese bekannten Carbodiimid-Methode. Beispielhaft wird hier Cyclohexylcarbodiimed (DCC) als Kopplungsreagenz angegeben. Nach der Etablierung einer Peptidbindung ist die Hyaluronsäure kovalent an die Elektrodenoberfläche, hier speziell die Iridiumnitrid-Beschichtung 12, gebunden. Geeignete Verfahrenparameter und -varianten zur Herstellung von Beschichtungen dieses Typs können unter anderem auch der US 5,527,893 und 5,585,361 - deren Offenbarung hiermit ausdrücklich eingeschlossen wird - entnommen werden.

### Ausführungsbeispiel 2 - Tauchbeschichtung

Neben der kovalenten Anbindung, können auch durch einfache Tauchbeschichtung Hyaluronsäure und/oder Hyaluronsäure-Derivate auf die Elektrodenoberfläche aufgebracht werden.

Die Elektrodenoberfläche wurde vorgereinigt und entfettet und unter leichtem Rühren für 10 Minuten bei Raumtemperatur in eine wässrige Lösung von Hyaluronsäure mit einem Molekulargewicht von mindestens 1.000.000 g/mol gelegt. Nach Entnahme und Trocknung wurde die Elektrode für mindestens 2 h bei ca. 30°C bis 40°C in eine Vernetzerlösung von 2 bis 4 ml Glutaraldehyd in einem Wasser-Aceton Gemisch getaucht. Danach wurde die Vernetzerlösung für mindestens weitere 2 h ausgetauscht. Anschließend wurde die Elektrode mehrfach mit destilliertem Wasser gespült und mit einer verdünnten Lösung von Natriumcyanoborhydrid reduktiv fixiert sowie mehrfach mit deionisiertem Wasser gespült. Nach Entnahme wurde die Probe für 24 Stunden bei 50°C im Trockenschrank getrocknet.

Das Molekulargewicht der Hyaluronsäure soll über 1.000.000 g/mol betragen, da die Hyaluronsäureketten durch die Sterilisation gespalten wird. Nach vorliegenden Untersuchungen kommt es bei einer Sterilisation mit Hilfe von Ethylenoxid oder beta-Bestrahlung (Elektronenbesschleuniger: 4,5 mEV, 25 kGy) zu 1 bis 2 Spaltungen pro Kette, d.h. native Hyaluronsäure liegt nach Sterilisation mit einem Molekulargewicht in der Größenordnung von 400.000 g/mol vor.

In Abhängigkeit von der Konzentration der wässrigen Hyaluronsäurelösung konnten folgende Schichtdicken erzielt werden:
(a) bei 0.25%iger wässriger Hyaluronsäurelösung: 90µm,
(b) bei 0,5%iger wässriger Hyaluronsäurelösung: 160µm,
(c) bei einer 1%igen wässrigen Hyaluronsäurelösung: 200µm
(d) und bei einer 2%igen wässrigen Hyaluronsäurelösung: 145µm.

### Ausführungsbeispiel 3 - Chitosan als Haftvermittler

Die Elektrodenoberfläche wurde vorgereinigt, entfettet und unter leichtem Rühren für 10 Minuten bei Raumtemperatur in eine 0,5 bis 2%ige Essigsäure mit einer Chitosankonzentration zwischen 0,1% und 0,5% gerührt. Das Molekulargewicht des Chitosans betrug zwischen 100.000 g/mol und 1.000.000 g/mol. Anschließend wurde die Elektrode entnommen und getrocknet.

Alternativ konnte eine dünne Schicht aus Chitosan durch Aufsprühen auf die Elektroden aufgebracht werden. Hierzu wurde eine 0,5%ige Chitosanlösung in einer 0,5%igen Essigsäure angesetzt. Die vorgereinigten Elektroden wurden 5 bis 20 mal im Abstand von 15 bis 30 Sekunden für 0,5 bis 1,0 sec mit Hilfe einer Airbrushpistole besprüht, wobei zwischen den Sprühschritten die Elektroden bei 40°C bis 70°C getrocknet wurden. Die aufgebrachten Schichten wiesen eine Schichtdicke von 1 µm bis 10µm auf.

Nach Trocknung wurde die Elektrode unter leichtem Rühren für 10 Minuten bei Raumtemperatur in eine wässrige Lösung von Hyaluronsäure mit einem Molekulargewicht von mindestens 1.000.000 g/mol gelegt. Nach Entnahme und Trocknung wurde die Elektrode für mindestens 2 h bei ca. 30°C bis 40°C in eine Vernetzerlösung von 2 bis 4 ml Glutaraldehyd in einem Wasser-Aceton Gemisch getaucht. Danach wurde die Vernetzerlösung ausgetauscht und die Vernetzung 2 h fortgeführt. Die Versuchsbedingungen führen auch zu einer Vernetzung von Chitosan mit dem Glutaraldehyd. Die säurekatalysierte Reaktion des Aldehyds mit dem Amin des Chitosans findet unter Bildung einer Schiffschen Base statt.

Anschließend wurde die Elektrode mehrfach mit destilliertem Wasser gespült und mit einer verdünnten Lösung von Natriumcyanoborhydrid reduktiv fixiert sowie mehrfach mit deionisiertem Wasser gespült. Die Nachbehandlung führt zur Reduktion der Schiffschen Base und freier Aldehydfunktionen. Nach Entnahme wurde die Probe für 24 Stunden bei 50°C im Trockenschrank getrocknet.

Das Chitosan fungiert als Haftvermittler, da Chitosan selbst im neutralen Bereich (Blut) schwer löslich ist. Zudem liegt das Chitosan vernetzt vor und bildet durch die Vernetzung mit Hilfe des Glutaraldehyd auch einen kovalenten Verbund zur aufgebrachten Hyaluronsäureschicht. Die dünne Haftvermittlerschicht aus Chitosan von 0,1 µm bis 50 µm, vorzugsweise von 1 µm bis 10 µm, hat keine signifikante Beeinträchtigung der elektrischen Übertragungseigenschaften der Elektrode zur Folge.

### Ausführungsbeispiel 4 - Chitosan als Zusatz

Neben den Polyanionen Hyaluronsäure bzw. seinen Hyaluronsäure-Derivaten kann die Schicht noch Polykationen wie Chitosan enthalten. Durch das Amin des Chitosans liegt eine weitere funktionelle Gruppe für den Vernetzer Glutardialdehyd vor. Die Aldehydfunktion kann sowohl mit der Aminfunktion des Chitosans als auch mit der Carbonyl- bzw. Hydroxylfunktion der Hyaluronsäure reagieren. Durch diese Reaktionen kann der Vernetzungsgrad zusätzlich erhöht und die ionische Wechselwirkung zwischen den Polyanionen und Polykationen zusätzlich verstärkt werden. Das Schichtsystem aus Polyanionen und Polykationen kann durch abwechselndes Besprühen der Elektroden mit Lösungen gewünschter Konzentrationen von Chitosan, Hyaluronsäure und Hyaluronsäure-Derivaten hergestellt werden.

Hierbei werden vorgereinigte Elektroden abwechselnd mit einer wässrigen Lösung aus Hyaluronsäure oder Hyaluronsäure-Derivat und in Essigsäure gelöstem Chitosan besprüht. Dabei beträgt die Konzentration der Hyaluronsäure oder Hyaluronsäure-Derivate 0,1% bis 1%, vorzugsweise 0,2% bis 0,5%. Die Konzentration der Essigsäure beträgt 0,1% bis 2%, vorzugsweise 0,5% bis 1%. Die Konzentration des Chitosans beträgt 0,1% bis 1%, vorzugsweise 0,2% bis 0,5%. Das Molekulargewicht der Hyaluronsäure oder der Hyaluronsäure-Derivate beträgt mindestens 1.000.000 g/mol und das Molekulargewicht des Chitosans mindestens 100.000 g/mol. Beide Lösungen werden im Abstand von 2 Sekunden bis 60 Sekunden, vorzugsweise 15 Sekunden bis 30 Sekunden, mit Hilfe eines Sprühverfahrens abwechselnd auf die Elektroden aufgebracht.

Durch die Wahl der Konzentration an Hyaluronsäure bzw. Chitosan und der jeweiligen Sprühdauer kann der jeweilige Anteil an Polyanionen und Polykationen eingestellt werden. Der Gewichtsanteil an Chitosan am gesamten Schichtsystem beträgt nicht mehr als 50%. Die Anzahl der Sprühschritte bestimmt die Schichtdicke des gesamten Schichtsystems. So werden bei 60 Sprühschritten mit einer Sprühdauer von 0,5 Sekunden mit üblichen Airbrushpistolen Schichtdicken zwischen 5 µm und 10 µm, gemessen im trockenen Zustand, erreicht. Nach der Beschichtung wird die Elektrode getrocknet und anschließend für mindestens 2 h bei ca. 30°C bis 40°C in eine Vernetzerlösung von 2 bis 4 ml Glutaraldehyd in einem Wasser-Aceton Gemisch getaucht. Danach wird die Vernetzerlösung für mindestens weitere 2 h ausgetauscht. Anschließend wird die Elektrode mehrfach mit destilliertem Wasser gespült und mit einer verdünnten Lösung von Natriumcyanoborhydrid reduktiv fixiert, sowie mehrfach mit deionisiertem Wasser gespült. Nach Entnahme wird die Probe für 24 Stunden bei 50°C im Trockenschrank getrocknet.

Die elektrischen Übertragungseigenschaften der Elektrode werden - bis zu einer maximalen Schichtdicke von 400 µm - nicht signifikant beeinträchtig.

### Einbindung eines therapeutischen Wirkstoffs

Ergänzend zur bereits eigenständigen therapeutischen Wirkung der Hyaluronsäure können entzündungshemmende Steroide, wie Dexamethason bzw. Dexamethasonnatriumphosphat (DMNP), in die Beschichtung eingebettet werden, die durch den allmählichen Abbau der Beschichtung bzw. Diffusion in das umliegende Gewebe abgegeben werden.

Die Darstellung der Polysaccharidschicht erfolgt in gleicher Weise wie in Beispiel 2 beschrieben, jedoch wird vor dem Trocknen die Elektrode mit 2 bis 4ml einer Lösung vom 50 mg/ml DMNP für eine Stunde gespült.

Ohne weitere Spülschritte erfolgt dann die Trocknung, wie in Beispiel 2 beschrieben.

### Untersuchungen zum Quellverhalten

Ein unterschiedlicher Vernetzungsgrad führt bei ansonsten gleicher reduktiver Fixierung zu einem unterschiedlichen Quellverhalten der Hyaluronsäure. Das Quellverhalten lässt sich u.a. gravimetrisch bestimmen. Weiterhin lässt sich der Vernetzungsgrad auch durch infrarotspektroskopische Analyse an vernetzten Hyaluronsäurefolien bestimmen. Der Bezug zur Degradation kann durch eine GPC Analytik, d.h. durch Molmassenbestimmung degradierter Hyaluronsäure, an Eluenten hergestellt werden.

Um den Einfluss von Vernetzungsparametern auf die Vernetzung und damit auch auf das Quellverhalten zu bestimmen, wurden die Parameter Temperatur, Wassergehalt, Art des Vernetzers und die Vernetzungsdauer variiert. Zur Bestimmung der Korrelation zwischen Quellverhalten und den Vernetzungsparametern wurden Hyaluronsäurefolien gegossen und vernetzt.

### Beispiele 1 bis 8 - Versuche zum Quellverhalten

Das Verfahren nach Beispiel 1 gliederte sich in folgende Schritte:
(a) Ansetzen einer 1 %igen Hyaluronsäurelösung;
(b) Ausgießen von 3 ml 1%iger Hyaluronsäurelösung in Petrischalen mit 4 cm Durchmesser und anschließendes Trocknen;
(c) Zugabe von 4 ml Vernetzerlösung zu den Folien bei Raumtemperatur (20°C), wobei die Vernetzerlösung aus 240 ml Aceton, 80 ml 25%ige Glutaraldehydlösung und 1,6 ml 3 molare Salzsäure bestand;
(d) Vernetzungsdauer 20 Stunden, wobei die Vernetzerlösung nach 4 Stunden ausgetauscht wurde;
(e) Entnahme und Spülen mit deionisiertem Wasser;
(f) Zugabe von 4 ml 2,2 %ige NaBH₃CN-Lösung;
(g) Spülen mit deionisiertem Wasser;
(h) Trocknen.

Die weiteren Beispiele 2 bis 8 wichen bei sonst gleicher Verfahrensführung wie folgt ab:
In Beispiel 2 betrug die Vernetzungsdauer im Schritt (d) 4 h ohne Wechsel der Vernetzerlösung.
In Beispiel 3 betrug die Vernetzungsdauer im Schritt (d) 2 h ohne Wechsel der Vernetzerlösung.
In Beispiel 4 enthielt die in Schritt (c) genannte Vernetzerlösung zusätzlich 20 ml deionisiertes Wasser.
In Beispiel 5 enthielt die in Schritt (c) genannte Vernetzerlösung zusätzlich 100 ml deionisiertes Wasser.
In Beispiel 6 enthielt die in Schritt (c) genannte Vernetzerlösung 80 ml 25%ige Formaldehydlösung anstelle der Glutaraldehydlösung.
In Beispiel 7 wurde die Vernetzung in Schritt (d) bei 30°C durchgeführt und die Vernetzungsdauer im Schritt (d) betrug 6,5 h, wobei nach 1,5 h die Vernetzerlösung ausgetauscht wurde.
In Beispiel 8 wurde die Vernetzung in Schritt (d) bei 30°C durchgeführt und die Vernetzungsdauer im Schritt (d) betrug 7 h, wobei nach 2 h die Vernetzerlösung ausgetauscht wurde.

Nach Trocknen der vernetzten Folien wurden diese gewogen und anschließend in deionisiertem Wasser für 30 Minuten gespült, kurz abgetupft und erneut gewogen, um das Quellverhalten, welches mit dem Vernetzungsgrad korreliert, zu bestimmen.

Die ermittelten Quellfaktoren lassen sich der nachfolgenden Tabelle entnehmen:

**Tabelle 1 - Quellfaktoren**

| | | | | | | | | |
|---|---|---|---|---|---|---|---|---|
| Beispiel | 1 | 2 | 3 | 4 | 5 | 6 | 7 | 8 |
| Quellfaktor | 6 | 14 | 75 | 7 | 7 | 34 | 10 | 13 |

Die beispielhaft aufgeführten Versuche zur Vernetzung lassen folgende Schlussfolgerungen zu:
Die Vernetzungsdauer hat einen signifikanten Einfluss auf den Vernetzungsgrad, was sich im Quellverhalten nieder schlägt. Bei einer Vernetzungsdauer von nur 2 Stunden werden Hyaluronsäurefolien erhalten, welche instabil sind und sich innerhalb weniger Stunden in Wasser auflösen. Hingegen werden bei einer Vernetzungsdauer von 4 Stunden stabile Hyaluronsäurefolien erhalten, welche aber gegenüber den Folien des Standardverfahrens einen höheren Quellfaktor zeigen. Der Wassergehalt der Vernetzerlösung hat im untersuchten Bereich keinen starken Einfluss auf den Quellfaktor und somit den Vernetzungsgrad. Die Verwendung von Formaldehyd anstelle von Glutaraldehyd führt zu vernetzten Hyaluronsäurefolien mit einem wesentlich höheren Quellfaktor. Dies lässt sich vermutlich auf die kürzere Kettenlänge des Formaldehyds zuzurückführen. Der kürzere Vernetzer Formaldehyd führt somit zu leicht vernetzten Hyaluronsäurefolien. Eine Vernetzung bei Temperatur von 30°C führt zu Hyaluronsäurefolien mit einem etwas höheren Quellfaktor und somit geringeren Vernetzungsgrad.

## Patentansprüche

1. Implantierbare Stimulationselektrode (10) zur Verwendung mit einem implantierbaren Gewebsstimulator, insbesondere Herzschrittmacher, Defibrillator, Knochen- oder Neurostimulator, wobei die Stimulationselektrode (10) eine gewendelte Zuleitung (13) mit einer elektrisch isolierenden Ummantelung (14) und einen metallischen Grundkörper (11), welcher am distalen Ende der Zuleitung befestigt ist und auf dem eine oder mehrere Zwischenschichten (12) aus Iridiumnitrid (IrN) mit einer fraktalartig vergrößerten Oberfläche aufgebracht sind, umfasst,
wobei die Zwischenschicht(en) (12) mit einer Polysaccharidschicht aus Hyaluronsäure und/oder Hyaluronsäure-Derivaten (17) mit einer Schichtdicke zwischen 10 und 400 µm bedeckt sind.

2. Stimulationselektrode nach Anspruch 1, wobei die Hyaluronsäure und Hyaluronsäure-Derivate nach einer Sterilisation ein durchschnittliches Molekulargewicht zwischen 300.000 bis 500.000 Dalton aufweisen.

3. Stimulationselektrode nach Anspruch 2, wobei das durchschnittliche Molekulargewicht zwischen 380.000 bis 420.000 Dalton liegt.

4. Stimulationselektrode nach einem oder mehreren der vorhergehenden Ansprüche, wobei die Polysaccharidschicht derart beschaffen ist, dass die in vivo Degradation der Polysaccharidschicht von außen in Richtung des Grundkörpers (11) der Stimulationselektrode (10) verlangsamt ist.

5. Stimulationselektrode nach Anspruch 4, wobei ein innerer Bereich der Polysaccharidschicht zumindest nicht vollständig innerhalb von zwei Jahren in vivo abbaubar ist.

6. Stimulationselektrode nach Anspruch 5, wobei der innere Bereich 3 bis 50 µm, insbesondere 5 bis 20 µm, dick ist.

7. Stimulationselektrode nach Anspruch 4, wobei ein äußerer Bereich der Polysaccharidschicht innerhalb von 100 Tagen in vivo abbaubar ist.

8. Stimulationselektrode nach Anspruch 7, wobei der äußere Bereich 10 bis 250 µm, insbesondere 50 bis 150 µm, dick ist.

9. Stimulationselektrode nach Anspruch 4, wobei die Polysaccharidschicht zumindest zwei Teilschichten mit unterschiedlichem Degradationsverhalten umfasst, wobei das Degradationsverhalten innerhalb jeder Teilschicht kontinuierlich veränderlich oder konstant über die Teilschicht festlegbar ist.

10. Stimulationselektrode nach Anspruch 9, wobei die Polysaccharidschicht eine innere Teilschicht umfasst, die um nicht mehr als 20 Gew% innerhalb von 2 Jahren in vivo abbaubar ist.

11. Stimulationselektrode nach Anspruch 10, wobei die innere Teilschicht 3 bis 50 µm, insbesondere 5 bis 20 µm, dick ist.

12. Stimulationselektrode nach Anspruch 9, wobei die Polysaccharidschicht eine äußere Teilschicht umfasst, die zumindest um mehr als 50 Gew% innerhalb von 100 Tagen in vivo abbaubar ist.

13. Stimulationselektrode nach Anspruch 12, wobei die äußere Teilschicht 10 bis 250 µm, insbesondere 50 bis 150 µm, dick ist.

14. Stimulationselektrode nach Anspruch 13, wobei die Schichtdicke 50-120 µm beträgt.

15. Stimulationselektrode nach einem oder mehreren der vorhergehenden Ansprüche, wobei die Beschichtung (17) Dexamethason und/oder Dexamethasonnatriumphosphat (DMNP) in einer zur Entfaltung einer pharmakologischen Wirkung ausreichenden Konzentration enthält.

16. Stimulationselektrode nach einem oder mehreren der vorhergehenden Ansprüche, wobei die Hyaluronsäure oder Hyaluronsäure-Derivate als Einzelsubstanzen, Co- oder Blockpolymere aus Hyaluronsäure und Hyaluronsäure-Derivaten oder in Form von Mischungen der vorgenanten Einzelsubstanzen und Polymere Bestandteil der Beschichtung (17) sind.

17. Stimulationselektrode nach einem oder mehreren der vorhergehenden Ansprüche, wobei die Polysaccharidschicht kovalent oder durch Physisorption an der Stimulationselektrodenoberfläche immobilisiert ist.

18. Stimulationselektrode nach einem oder mehreren der vorhergehenden Ansprüche, wobei die Polysaccharidschicht eine Haftvermittlerschicht aus Chitosan umfasst.

19. Stimulationselektrode nach Anspruch 18, wobei die Haftvermittlerschicht 0,1 bis 50 µm, insbesondere 1 bis 10 µm, dick ist.

20. Stimulationselektrode nach einem oder mehreren der vorhergehenden Ansprüche, wobei die Polysaccharidschicht zumindest in Teilbereichen oder Teilschichten Chitosan beinhaltet.

21. Stimulationselektrode nach Anspruch 20, wobei ein Anteil des Chitosans am Gesamtgewicht der Polysaccharidschicht nicht mehr als 50 Gew.% beträgt.

## Claims

1. An implantable stimulation electrode (10) for use with an implantable tissue stimulator, in particular a cardiac pacemaker, defibrillator, bone stimulator or neurostimulator, wherein the stimulation electrode (10) comprises a coiled feed line (13) having an electrically insulating sheath (14) and a metallic base body (11), which is fastened to the distal end of the feed line and to which one or more intermediate layers (12) made of iridium nitride (IN) having a fractal-like increased surface are applied,
wherein the intermediate layer or layers (12) are covered with a polysaccharide layer comprising hyaluronic acid and/or hyaluronic acid derivatives (17) having a layer thickness between 10 and 400 µm.

2. The stimulation electrode according to claim 1, wherein the hyaluronic acid and hyaluronic acid derivatives have an average molecular weight between 300,000 and 500,000 dalton after sterilization.

3. The stimulation electrode according to claim 2, wherein the average molecular weight ranges between 380,000 and 420,000 dalton.

4. A stimulation electrode according to any one or more of the preceding claims, wherein the polysaccharide layer is designed such that the in vivo degradation of the polysaccharide is slowed from the outside in the direction of the base body (11) of the stimulation electrode (10).

5. The stimulation electrode according to claim 4, wherein an inner region of the polysaccharide layer cannot be decomposed in vivo within two years, at least not entirely.

6. The stimulation electrode according to claim 5, wherein the inner region is 3 to 50 µm, and more particularly 5 to 20 µm, thick.

7. The stimulation electrode according to claim 4, wherein an outer region of the polysaccharide layer can be decomposed in vivo within 100 days.

8. The stimulation electrode according to claim 7, wherein the outer region is 10 to 250 µm, and more particularly 50 to 150 µm, thick.

9. The stimulation electrode according to claim 4, wherein the polysaccharide layer comprises at least two partial layers having different degradation behaviors, wherein the degradation behavior within each partial layer can be set to be continuously variable or constant over the partial layer.

10. The stimulation electrode according to claim 9, wherein the polysaccharide layer comprises an inner partial layer, which cannot be decomposed in vivo within 2 years by more than 20% by weight.

11. The stimulation electrode according to claim 10, wherein the inner partial layer is 3 to 50 µm, and more particularly 5 to 20 µm, thick.

12. The stimulation electrode according to claim 9, wherein the polysaccharide layer comprises an outer partial layer, which can be decomposed in vivo within 100 days by at least more than 50% by weight.

13. The stimulation electrode according to claim 12, wherein the outer partial layer is 10 to 250 µm, and more particularly 50 to 150 µm, thick.

14. The stimulation electrode according to claim 13, wherein the layer thickness is 50 to 120 µm.

15. A stimulation electrode according to any one or more of the preceding claims, wherein the coating (17) comprises dexamethasone and/or dexamethasone sodium phosphate (DSP) in a concentration that is sufficient for having a pharmacological effect.

16. A stimulation electrode according to any one or more of the preceding claims, wherein the hyalonuric acid or hyalonuric acid derivatives are constituents of the coating (17) as individual substances, copolymers or block polymers comprising hyalonuric acid and hyalonuric acid derivatives, or in the form of mixtures of the above-mentioned individual substances and polymers.

17. A stimulation electrode according to any one or more of the preceding claims, wherein the polysaccharide layer is covalently immobilized or immobilized by physisorption on the stimulation electrode surface.

18. A stimulation electrode according to any one or more of the preceding claims, wherein the polysaccharide layer comprises an adhesion promoter layer comprising chitosan.

19. The stimulation electrode according to claim 18, wherein the adhesion promoter layer is 0.1 to 50 µm, and more particularly 1 to 10 µm, thick.

20. A stimulation electrode according to any one or more of the preceding claims, wherein the polysaccharide layer contains chitosan at least in partial regions or partial layers.

21. The stimulation electrode according to claim 20, wherein a content of chitosan of the total weight of the polysaccharide layer is no more than 50% by weight.

## Revendications

1. Électrode de stimulation implantable (10) destinée à être utilisée avec un stimulateur de tissus implantable, en particulier un stimulateur cardiaque, un défibrillateur, un stimulateur osseux ou un neurostimulateur, l'électrode de stimulation (10) comprenant une ligne d'alimentation torsadée (13) avec une enveloppe électriquement isolante (14) et un corps de base métallique (11), qui est fixé à l'extrémité distale de la ligne d'alimentation et sur lequel sont appliquées une ou plusieurs couches intermédiaires (12) de nitrure d'iridium (IrN) avec une surface agrandie de manière fractale,
dans laquelle la ou les couches intermédiaires (12) sont revêtues d'une couche de polysaccaride à base d'acide hyaluronique et/ou de dérivés d'acide hyaluronique (17) avec une épaisseur de couche entre 10 et 400 µm.

2. Électrode de stimulation selon la revendication 1, dans laquelle l'acide hyaluronique et les dérivés d'acide hyaluronique présentent un poids moléculaire moyen entre 300.000 et 500.000 Dalton après stérilisation.

3. Électrode de stimulation selon la revendication 2, dans laquelle le poids moléculaire moyen est compris entre 380.000 et 420.000 Dalton.

4. Électrode de stimulation selon l'une ou plusieurs des revendications précédentes, dans laquelle la couche de polysaccaride est réalisée de telle manière, que la dégradation in vivo de la couche de polysaccaride est ralentie de l'extérieur dans la direction du corps de base (11) de l'électrode de stimulation (10).

5. Électrode de stimulation selon la revendication 4, dans laquelle une région interne de la couche de polysaccaride est dégradable in vivo en l'espace de deux ans, du moins pas complètement.

6. Électrode de stimulation selon la revendication 5, dans laquelle la région interne présente une épaisseur de 3 à 50 µm, en particulier de 5 à 20 µm.

7. Électrode de stimulation selon la revendication 4, dans laquelle une région extérieure de la couche de polysaccaride est dégradable in vivo en l'espace de 100 jours.

8. Électrode de stimulation selon la revendication 7, dans laquelle la région extérieure présente une épaisseur de 10 à 250 µm, en particulier de 50 à 150 µm.

9. Électrode de stimulation selon la revendication 4, dans laquelle la couche de polysaccaride comprend au moins deux couches partielles avec des comportements de dégradation différents, le comportement de dégradation dans chaque couche partielle pouvant être modifié en continu ou alors constant sur l'ensemble de la couche partielle.

10. Électrode de stimulation selon la revendication 9, dans laquelle la couche de polysaccaride comprend une couche partielle intérieure dégradable in vivo à maximum 20% en poids en l'espace de 2 ans.

11. Électrode de stimulation selon la revendication 10, dans laquelle la couche partielle intérieure présente une épaisseur de 3 à 50 µm, en particulier de 5 à 20 µm.

12. Électrode de stimulation selon la revendication 9, dans laquelle la couche de polysaccaride comprend une couche partielle extérieure dégradable in vivo à au moins 50% en poids en l'espace de 100 jours.

13. Électrode de stimulation selon la revendication 12, dans laquelle la couche partielle extérieure présente une épaisseur de 10 à 250 µm, en particulier de 50 à 150 µm.

14. Électrode de stimulation selon la revendication 13, dans laquelle l'épaisseur de couche est comprise entre 50 et 120 µm.

15. Électrode de stimulation selon l'une ou plusieurs des revendications précédentes, dans laquelle le revêtement (17) contient de la dexaméthasone et/ou du phosphate de sodium de dexaméthasone (DMNP), avec une concentration suffisante pour le déploiement d'un effet pharmacologique.

16. Électrode de stimulation selon l'une ou plusieurs des revendications précédentes, dans laquelle l'acide hyaluronique ou les dérivés d'acide hyaluronique sont composants du revêtement (17) sous la forme de substances individuelles, de copolymères ou à blocs, à base d'acide hyaluronique et de dérivés d'acide hyaluronique, ou encore sous forme de mélanges des substances individuelles citées avec des polymères.

17. Électrode de stimulation selon l'une ou plusieurs des revendications précédentes, dans laquelle la couche de polysaccaride est covalente ou immobilisée par physisorption à la surface de l'électrode de stimulation.

18. Électrode de stimulation selon l'une ou plusieurs des revendications précédentes, dans laquelle la couche de polysaccaride comprend une couche d'agents adhésifs à base de chitosan.

19. Électrode de stimulation selon la revendication 18, dans laquelle la couche d'agent adhésif présente une épaisseur de 0,1 à 50 µm, en particulier de 1 à 10 µm.

20. Électrode de stimulation selon l'une ou plusieurs des revendications précédentes, dans laquelle la couche de polysaccaride contient du chitosan au moins dans des régions partielles ou dans des couches partielles.

21. Électrode de stimulation selon la revendication 20, dans laquelle la part de chitosan dans le poids global de la couche de polysaccaride n'est pas supérieure à 50% en poids.
